# EUROPÄISCHE PATENTSCHRIFT

(11) **EP 3 330 728 B1**
(45) Veröffentlichungstag und Bekanntmachung des Hinweises auf die Patenterteilung: **21.10.2020**
(21) Anmeldenummer: 17172146.7
(22) Anmeldetag: 22.05.2017
(51) Int. Cl.: G01R 33/3875, G01R 33/56, G01R 33/563

(54) **VERFAHREN ZUR GEFÄSSDARSTELLUNG MIT HILFE EINER MR-ANLAGE**
METHOD FOR VASCULAR IMAGING WITH THE AID OF AN MR EQUIPMENT
PROCÉDÉ D'IMAGERIE VASCULAIRE À L'AIDE D'UN ÉQUIPEMENT RM

(43) Veröffentlichungstag der Anmeldung: 06.06.2018
(73) Patentinhaber: Siemens Healthcare GmbH, 91052 Erlangen (DE)
(72) Erfinder: Speier, Peter, 91056 Erlangen (DE)

(56) Entgegenhaltungen:
- JP-A- 2005 152 534
- US-A- 5 557 202

## Beschreibung

Die vorliegende Erfindung betrifft ein Verfahren zur Gefäßdarstellung mit Hilfe einer Magnet-Resonanz (MR)-Anlage basierend auf der Time-of-Flight (TOF)-Technik. Weiter werden die zugehörige MR-Anlage, ein Computerprogrammprodukt und ein elektronisch lesbarer Datenträger bereitgestellt.

Eine Klasse von MR-Bildgebungverfahren, insbesondere Perfusions- und Angiographiemessungen mit der TOF-Technik, aber auch einige 2D-Herzbildgebungsverfahren, verwendet einen zuflussbasierten Kontrast. Bei letzteren verstärkt der Zufluss lediglich bereits den vorhandenen Kontrast der Gefäßwände und vereinfacht so deren Darstellung, während Perfusions- und Angiographiemessungen allein auf zuflussbasiertem Kontrast basieren. In dem Fall, dass quantitative Zuflussinformationen extrahiert werden sollen, wie etwa bei Perfusionsmessungen, wird ein Differenzbildgebungsverfahren angewandt, um das Hintergrundsignal zu eliminieren, d.h. eine Subtraktion zweier Datensätze mit unterschiedlichen Zufluss-Magnetisierungsvorbereitungen wird durchgeführt. Dieser Ansatz verdoppelt jedoch die Messzeit und erfordert zudem eine Registrierung der beiden Datensätze zueinander. Daher wird dieser Schritt in Fällen übersprungen, in denen quantitative Informationen nicht gebraucht werden, z.B. bei einer Bildgebung der vaskulären Geometrie. Stattdessen wird das Hintergrundsignal soweit möglich mit Hilfe von MR-Verfahren unterdrückt.

Die MR-Bildgebung basierend auf der TOF-Technik ist ein nicht invasives Bildgebungsverfahren zur Darstellung der Gefäßstruktur eines Untersuchungsobjekts, welches auf dem Hineinfließen von "frischen" nicht vorgesättigten Spins in ein Bildgebungsvolumen beruht. Die ortsfeste Magnetisierung, d.h. die ortsfesten Spins der Bildgebungsebene werden durch wiederholte Anregung in einem kurzen Zeitabstand gesättigt. Das Signal dieser Magnetisierung ist weitgehend unterdrückt, während die nicht vorgesättigte Magnetisierung, die sich beispielsweise durch den Blutfluss während der Aufnahme in die Bildgebungsebene hinein ergibt, einen hohen Signalanteil aufweist.

Für die MR-Bildgebung basierend auf der TOF-Technik des Kopfgefäßsystems sind insbesondere die im folgenden beschriebenen Verfahren zum Unterdrücken der von Gehirngewebe, Spinalflüssigkeit und Fett verursachten Hintergrundsignale bekannt.

Ein erstes bekanntes Verfahren zum Unterdrücken der Hintergrundsignale ist das Anwenden hoher Kippwinkel bei der Bildaufnahme, um Gewebe mit langen T1-Zeiten, insbesondere Flüssigkeiten, zu unterdrücken. Der Kippwinkel ist durch die Tatsache eingeschränkt, dass er das Signal mit jedem Puls verringert, daher begrenzt er die Distanz, über die zufließende Spins in dem Bildgebungsvolumen beobachtet werden können, d.h. die Schichtdicke des Bildgebungsvolumens. Typischerweise ermöglichen Kippwinkel im Bereich von 15 Grad/24 ms eine gute Hintergrundsignalunterdrückung bei Schichtdicken von bis zu 2 cm.

Ein weiteres bekanntes Verfahren zur Unterdrückung von Hintergrundsignalen ist das Opposed-Phase-Imaging-Verfahren. Dabei wird die Echozeit (TE) derart gewählt, dass Fett und Wasser nicht phasengleich (dephasiert) sind, z.B. ungefähr 7 ms bei 1,5 T und 3,4 ms bei 3 T. Dadurch werden Signale von Voxel, welche eine Wasser-Fett-Mischung enthalten, reduziert. Allerdings sind Voxel mit reinem Fett immer noch hell sichtbar und beeinträchtigen die Gefäßsichtbarkeit in diesen Bereichen oder im Allgemeinen in nichtselektiven Maximumintensitätsprojektionen (MIP). Insbesondere bei 1,5 T verlängert diese Bedingung die Echozeit, wodurch ein T2*-basierter Signalverlust und eine Verlängerung der Erfassungszeit verursacht wird. Um das Signal für derartige Echozeiten zu stabilisieren, müssen Flusskompensationsgradienten angewendet werden. Dabei wird ein Dephasieren aufgrund einer laminaren, geraden Strömung kompensiert, nicht jedoch ein Dephasieren aufgrund einer turbulenten Strömung oder einer Strömung entlang einer Krümmung. Daher wäre es vorteilhaft, die Echozeit zu minimieren, z.B. durch das Verwenden von Ultrashort-Echo-Time (UTE) -Techniken, und das Hintergrundsignal von Fett mit einem anderen Verfahren zu unterdrücken.

Ein weiteres bekanntes Verfahren zur Unterdrückung von Hintergrundsignalen ist die Sättigung durch eine Magnetisierungsübertragung (MT) mittels eines MT-Pulses. Dabei werden unbewegliche Protonen in Makromolekülen mit einer verbreiterten Resonanzfrequenz durch starke HF-Pulse mit Frequenzen in Bereichen, welche 500 Hz bis 2000 Hz neben der Wasserfrequenz liegen, gesättigt. Diese Protonen koppeln mit den beobachtbaren hochmobilen Wasserprotonen, wodurch eine Sättigung der unbeweglichen Protonen auch zu einer Sättigung des beobachteten Signals führt. Allerdings sind beobachtbare Fettprotonen nicht gleich stark an die unbeweglichen Protonen gekoppelt und deswegen kann das Fettsignal nicht durch dieses Verfahren unterdrückt werden. Die Sättigung kann durch Erhöhen der abgestrahlten HF-Energie, limitiert durch Specific-Absorption-Rate (SAR)-Grenzwerte, und durch Minimieren des Abstandes der Frequenz von der Resonanzfrequenz, limitiert durch die Sättigung des Bluts, verstärkt werden. Da der MT-Puls nichtselektiv ist, muss nicht nur die Sättigung des Bluts innerhalb des Bildgebungsvolumens, sondern auch die Sättigung entlang der zuführenden Gefäße, z.B. der Carotiden, berücksichtigt werden.

Aus der Druckschrift US 5,627,468 A und der Veröffentlichung MRM 32, 52-59, 1994 von Miyazaki et al. ist ein weiteres Verfahren zur Unterdrückung von Hintergrundsignalen bekannt, bei welchem ein sogenannter Slice-Selective-Off-Resonance-Sinc (SORS)-Puls mit einem linearen Gradientenfeld entlang der z-Achse eingesetzt wird. Das Verfahren umfasst eine erste Sequenz mit dem Anwenden des SORS-Pulses und eine zweite Sequenz zur Aufzeichnung von MR-Messdaten. Dabei wird die Frequenz des SORS-Pulses derart gewählt, dass die Wasserfrequenz oberhalb des Bildgebungsvolumens getroffen wird, so dass innerhalb des Bildgebungsvolumens keine Wasseranregung, sondern lediglich ein MT-Effekt stattfindet. Zusätzlich wird dadurch venöses Blut oberhalb des Bildgebungsvolumens gesättigt, so dass der SORS-Puls gleichzeitig zum Blockieren von Signalen aus venösem Blut dient. Durch das lineare Gradientenfeld vergrößert sich der Abstand zwischen dem Pulsfrequenzband und der Wasserfrequenz unterhalb des Bildgebungsvolumens linear mit dem Abstand zum Bildgebungsvolumen, wodurch die Sättigung des zufließenden Bluts aufgrund einer unzureichenden Frequenzbandbegrenzung des SORS-Pulses verringert wird. Durch eine geeignete Wahl der Polarität des SORS-Pulses kann das Verfahren auch für eine Fettsättigung verwendet werden, jedoch ist die Fettsättigung wegen dem linearen Gradienten entlang der Schichtselektionsrichtung bei leicht vom Optimum abweichenden Verfahrensparametern und bei leichten B0-Inhomogenitäten typischerweise inhomogen.

Weiterhin ist die sogenannte frequenzselektive Fettsättigung ein bekanntes Verfahren zur Unterdrückung von Hintergrundsignalen. Dabei wird durch einen frequenzselektiven, räumlich nicht selektiven HF-Puls die Fettmagnetisierung im Bildgebungsvolumen angeregt, ohne die Wassermagnetisierung im Bildgebungsvolumen anzuregen. Die transverse Magnetisierung der angeregten Fettmagnetisierung wird zusätzlich durch Gradient Spoiling ausgelöscht und das Wassersignal kann somit separat gemessen werden. Dennoch ist dieses Verfahren unzuverlässig, da es das Blut in den zuführenden Gefäßen sättigt, und dadurch manche Blutgefäße im Bildgebungsvolumen nicht aufzulösen sind. Diese unerwünschte Sättigung des Bluts in den zuführenden Gefäßen wird durch Inhomogenitäten des B0-Magnetfelds verursacht, insbesondere im Nackenbereich und dem oberen Brustkorb einer Untersuchungsperson, wobei insbesondere die Wasserfrequenz durch die Inhomogenitäten hin zu der nominellen Fettfrequenz verschoben wird, d.h. in Richtung hin zu niedrigeren Frequenzen.

Wegen der beschriebenen Nachteile der herkömmlichen Verfahren zur Unterdrückung von Hintergrundsignalen muss oft zusätzlich eine manuelle Nachbearbeitung des MR-Bildes durch Ausschneiden des perkutanen Fetts vor der Erzeugung von Maximumintensitätsprojektionen (MIP) durchgeführt werden, was zu einem erhöhten Zeit- und Personalaufwand, und damit erhöhten Untersuchungskosten führt.

MR-Verfahren, bei denen unter anderem auch Hintergrundsignale unterdrückt werden, sind beispielsweise in der DE 10 2015 205 694 B3, der US 20160266223 A1 und der US 20150272453 A1 offenbart.

Aus der Druckschrift US 5,557,202 A ist ein Verfahren zur Magnetresonanzbildgebung bekannt, wobei in einer ersten Sequenz ein Binomialpuls angelegt wird, und in einer zweiten Sequenz Magnetresonanzbilddaten gesammelt werden. Bei dem Anlegen des Binomialimpulses wird die Zentralfrequenz des Binomialimpulses gegenüber der Resonanzfrequenz der im Wasser enthaltenen Protonen um einen vorgegebenen Offsetbetrag zur Seite der hohen Frequenzen hin versetzt.

Aus der Druckschrift JP 2005-152534 A ist eine Vorrichtung zur Magnetresonanzbildgebung und ein Verfahren zur HF-Wellenerzeugung bekannt, wobei ein MT-Puls und CHESS-Puls ein Anregungsfrequenzband aufweisen, welches in der Lage ist, gebundenes Wasser und Fett eines Untersuchungsobjekts anzuregen. Der CHESS-Puls ist amplitudenmoduliert, so dass die Mittenfrequenz des Anregungsfrequenzbandes des MT-Pulses und die Mittenfrequenz des Anregungsfrequenzbandes des CHESS-Pulses um eine gewünschte Offsetfrequenz voneinander verschoben werden und die MT-Pulse und der durch Amplitudenmodulation erhaltene modulierte CHESS-Puls zu einem synthetischen Impuls synthetisiert werden.

Es besteht daher Bedarf nach einem verbesserten Verfahren zum Unterdrücken von Hintergrundsignalen bei der MR-Bildgebung basierend auf der TOF-Technik, welches Hintergrundsignale von Gehirngewebe, Spinalflüssigkeit und Fett mit hoher Zuverlässigkeit unterdrückt, welches einen hohen Kontrast der MR-Bilder und eine gute Erkennbarkeit von insbesondere kleinen Gefäßen bereitstellt, und welches dadurch eine effiziente und kostengünstige MR-Bildgebung ohne die Notwendigkeit von manuellem Nachbearbeiten der MR-Bilder ermöglicht.

Diese Aufgabe wird mit den Merkmalen der unabhängigen Ansprüche gelöst. In den abhängigen Ansprüchen sind weitere Ausführungsformen der Erfindung beschrieben.

Gemäß einem ersten Aspekt wird ein Verfahren zur Gefäßdarstellung basierend auf der Time-of-Flight (TOF)-Technik mit Hilfe einer MR-Anlage bereitbestellt. In einem ersten Schritt wird ein Magnetfeld an ein Bildgebungsvolumen und ein Zuflussvolumen, von welchem Flüssigkeit in das Bildgebungsvolumen eintritt, einer Untersuchungsperson angelegt. In einem weiteren Schritt wird das Bildgebungsvolumen durch einen HF-Puls, welcher eine Magnetisierungs-Transferfunktion und eine Fett-Sättigungsfunktion erfüllt, angeregt während das Magnetfeld angelegt ist. Der HF-Puls, welcher eine Magnetisierungs-Transferfunktion und eine Fett-Sättigungsfunktion erfüllt, kann genau einen HF-Puls umfassen. Das Bildgebungsvolumen kann auch durch einen HF-Präparationsblock angeregt werden. Ein HF-Präparationsblock kann einen oder mehrere HF-Pulse umfassen, welche eine Fett-Sättigungsfunktion, eine Magnetisierungs-Transferfunktion und eine TSAT-Funktion erfüllen. In einem zusätzlichen Schritt werden MR-Signale aus dem Bildgebungsvolumen zum Darstellen von Gefäßen gemessen. Dabei weist der HF-Puls eine Frequenzverteilung auf, deren Frequenzen im Wesentlichen niedriger als die Mittenfrequenz des Wassers im Bildgebungsvolumen sind, d.h. auf der Seite der Fettfrequenz liegen, und welche die Fettfrequenz im Bildgebungsvolumen umfassen. Weiter weist das Magnetfeld eine Magnetfeldverteilung auf, welche durch eine Funktion angenähert ist, die derart ausgebildet ist, dass sie einen Scheitelbereich mit im Wesentlichen keinem räumlichen Gradienten im Bildgebungsvolumen aufweist und einen höheren räumlichen Gradienten im Zuflussvolumen aufweist, so dass die Wasserfrequenz im Zuflussvolumen nicht von dem HF-Puls umfasst wird. Die Funktion, welche die Magnetfeldverteilung annähert, kann nicht-linear sein. Die Funktion, welche die Magnetfeldverteilung annähert, kann anstatt des Scheitelpunktes eines symmetrischen Gradienten, d.h. beispielsweise eines z2-Shims, auch einen Sattelpunkt eines antisymmetrischen Gradienten, d.h. beispielsweise eines z3-Shims, aufweisen.

Die Feldverteilung ist derart ausgebildet, dass die Mittenfrequenz des Wassers in dem Zuflussvolumen in Richtung höherer Frequenzen verschoben wird, wodurch sie durch den HF-Puls nicht mehr beeinflusst wird. Insbesondere kann die Feldverteilung des Magnetfeldes eine im Wesentlichen konstante Feldstärke innerhalb des Bildgebungsvolumens aufweisen. Insbesondere können die Feldschwankungen des Magnetfeldes innerhalb des Bildgebungsvolumens kleiner als der Frequenzabstand zwischen der Fettfrequenz und der Wasserfrequenz sein, wodurch eine homogene Fettsättigung ermöglicht wird. Eine Längsrichtung kann weiter derart definiert sein, dass sie in Richtung von Fuß zu Kopf einer Untersuchungsperson verläuft, wobei die Längsrichtung durch das Zentrum des Bildgebungsvolumens und das Zentrum des Zuflussvolumens verläuft, und wobei sie weiter herkömmlicherweise auch als z-Richtung bezeichnet wird. Die Feldverteilung des Magnetfeldes weist im Zuflussvolumen einen im Vergleich zum Bildgebungsvolumen höheren Feldstärkegradienten in Längsrichtung auf, d.h. die Feldstärke des Magnetfeldes steigt in z-Richtung nichtlinear an, und ist insbesondere angenähert durch eine mathematische Funktion mindestens zweiten Grades. Das Magnetfeld weist in Bezug zum B0-Feld eine Polarität auf, welche die Feldstärke des B0-Feldes ausserhalb des Bildgebungsvolumens, zumindest in der Richtung, aus der das Blut einströmt, d.h. zumindest im Zuflussvolumen, verringert. Dadurch wird die Mittenfrequenz des Wassers in Richtung weg von der Fettfrequenz verschoben, insbesondere hin zu höheren Frequenzen verschoben, und weiter insbesondere mit steigendem Abstand in Längsrichtung nichtlinear hin zu höheren Frequenzen verschoben.

Durch das erfindungsgemäße Verfahren werden bei der Gefäßdarstellung mit Hilfe einer MR-Anlage basierend auf der TOF-Technik Hintergrundsignale von Gehirngewebe, Spinalflüssigkeit und Fett zuverlässiger unterdrückt und es wird ein höherer Kontrast und eine bessere Erkennbarkeit von insbesondere kleinen Gefäßen in der Nähe des Schädels und der Augenhöhle in resultierenden MR-Bildern bereitstellt als bei herkömmlichen Verfahren zum Unterdrücken von Hintergrundsignalen. Weiter kann durch das erfindungsgemäße Verfahren die Anwendung Opposed-Phase-Imaging-Verfahrens zur Unterdrückung von Hintergrundsignalen unterbleiben, wodurch vorteilhaft eine Minimierung der TE im bestehenden Messprotokoll durchgeführt werden kann, und besonders vorteilhaft eine wesentlich schnellere Ultrashort-Echo-Time (UTE) -MR-Bildgebung mit hohem Kontrast und ohne Signallücken aufgrund von turbulenten Strömungen ermöglicht wird. Zudem entfällt durch das erfindungsgemäße Verfahren die Notwendigkeit von manuellem Nachbearbeiten der MR-Bilder, um perkutanes Fett manuell zu entfernen. Insbesondere wird die Gefäßsichtbarkeit von Inline SAG und COR MIPs verbessert, was wiederum die Notwendigkeit von manuellem "Schädelfreistellen", wie es typischer weise im Klinikalltag durchgeführt wird, verringert. Dadurch wird eine schnellere und effizientere MR-Bildgebung zur Gefäßdarstellung mit Hilfe einer MR-Anlage ermöglicht, welche einen geringen Zeit- und Personalaufwand für eine Untersuchung, und damit verringerte Untersuchungskosten aufweist im Vergleich zu herkömmlichen Verfahren zur Gefäßdarstellung mit Hilfe einer MR-Anlage.

Das Bildgebungsvolumen und das Zuflussvolumen können schichtförmig sein, wobei eine Längsachse senkrecht zu dem Bildgebungsvolumen und dem Zuflussvolumen ausgerichtet sein kann, und wobei entlang der Längsachse eine Schichtdicke des Zuflussvolumens gleich einem Vielfachen einer Schichtdicke des Bildgebungsvolumens sein kann.

Durch die Schichtform und die im Vergleich zum Zuflussvolumen geringere Schichtdicke des Bildgebungsvolumens wird der Kontrast des MR-Bildes bei der Gefäßdarstellung weiter erhöht.

Der Scheitelbereich der Funktion kann im Wesentlichen die gleiche Dicke haben, wie die Schichtdicke des Bildgebungsvolumens, wodurch die Hintergrundsignale aufgrund von Inhomogenitäten im Zuflussvolumen zuverlässiger verringert werden.

Die Schritte des Verfahrens können für mindestens ein weiteres Bildgebungsvolumen und mindestens ein weiteres Zuflussvolumen durchgeführt werden. Dabei kann das Verfahren weiter ein Anpassen der Magnetfeldverteilung an das mindestens eine weiteren Bildgebungsvolumen und das mindestens eine weitere Zuflussvolumen, derart, dass die angepasste Magnetfeldverteilung in dem mindestens einen weiteren Bildgebungsvolumen den Scheitelbereich aufweist und in dem mindestens einen weiteren Zuflussvolumen den höheren räumlichen Gradienten aufweist, umfassen.

Durch das Durchführen des Verfahrens für mehrere Bildgebungsvolumina und Zuflussvolumina werden die Zuverlässigkeit und der Kontrast der MR-Bildgebung weiter erhöht.

Das Anpassen der Magnetfeldverteilung kann ein lineares räumliches Verschieben der Magnetfeldverteilung umfassen, wodurch die Anpassung des Magnetfelds schnell und effizient durchgeführt werden kann.

Das Anpassen der Magnetfeldverteilung kann ein Ändern einer linearen Komponente der Magnetfeldverteilung umfassen, wodurch die Effizienz der Anpassung weiter erhöht wird.

Die mehreren Bildgebungsvolumina können sich zumindest teilweise räumlich überschneiden. Durch die räumliche Überschneidung der mehreren Bildgebungsvolumina ist das Verfahren weniger fehleranfällig und die Unterdrückung der Hintergrundsignale zuverlässiger.

Das Verfahren kann weiter ein Erzeugen des HF-Pulses durch Überlagern eines ersten HF-Pulses und eines zweiten HF-Pulses umfassen. Das Erzeugen eines HF-Pulses durch Überlagern eines ersten HF-Pulses und eines zweiten HF-Pulses, ermöglicht eine exaktere Anpassung des HF-Pulses an mehrere Funktionen, etwa einer Magnetisierungs-Transferfunktion und eine Fett-Sättigungsfunktion, und damit einer effizientere Unterdrückung der Hintergrundsignale.

Dabei kann der erste HF-Puls einen ersten Flip-Winkel und der zweite HF-Puls einen zweiten Flip-Winkel unterschiedlich zu dem ersten Flip-Winkel aufweisen, wodurch der HF-Puls zur Anregung unterschiedlichen Gewebes angepasst werde kann, so dass er etwa eine Magnetisierungs-Transferfunktion und eine Fett-Sättigungsfunktion erfüllen kann.

Der erste HF-Puls kann vorzugsweise einen Flip-Winkel von 20 bis 180 Grad, besonders vorzugsweise 90 Grad, und eine Frequenz im Wesentlichen gleich der Fettfrequenz im Bildgebungsvolumen aufweisen. Diese Werte sind besonders vorteilhaft für die Fett-Sättigungsfunktion des HF-Pulses. Falls das Messen der MR-Signale im Steady State genau in der Mitte zwischen den HF-Pulsen zur Fettsättigung durchgeführt wird, ist eine Inversion, d.h. 180 Grad, für ein vollständiges Auslöschen des Fettsignals optimal. Typischerweise werden jedoch durch SAR beschränkt kleinere Kippwinkel verwendet. Bei HF-Pulsabständen << T1 von Fett, wie in dem oben beschriebenen Verfahren, kann auch ein Kippwinkel << 90 Grad gute Fettsättigung erreichen.

Pro HF-Puls können mehrere Messungen von MR-Signalen aus dem Bildgebungsvolumen durchgeführt werden, wodurch die Messzeit für die Aufnahme eines MR-Bildes verringert wird.

Die mehreren Messungen können in gleichen zeitlichen Abständen durchgeführt werden, wodurch die Bildqualität der MR-Bilder verbessert wird.

Die mehreren Messungen können in Messgruppierungen mit zeitlichen Abständen zwischen den einzelnen Messungen durchgeführt werden, wobei zwischen den Messgruppierungen eine Anregung durch einen HF-Puls oder einen HF-Präparationsblock durchgeführt werden kann, und wobei der mittlere zeitliche Abstand zwischen den Messungen kleiner einem vorbestimmten Schwellenwert sein kann. Ein HF-Präparationsblock kann einen oder mehrere HF-Pulse umfassen, welche eine Fett-Sättigungsfunktion, eine Magnetisierungs-Transferfunktion und eine TSAT-Funktion erfüllen. Durch die zeitliche Gruppierung der einzelnen Messungen zu Messgruppierungen mit einem zwischen den Messgruppierungen ausgespielten HF-Puls wird die Bildqualität der MR-Bilder weiter verbessert, indem der HF-Puls ein wohldefiniertes Frequenzprofil aufweisen kann. Weiter wird die SAR Belastung für das Untersuchungsobjekt verringert.

Gemäß einem weiteren Aspekt der Erfindung wird eine MR-Anlage zur Gefäßdarstellung basierend auf der Time-of-Flight (TOF)-Technik bereitgestellt, wobei die MR-Anlage eine MR-Steuereinheit und eine Speichereinheit aufweist, wobei die Speichereinheit von der MR-Steuereinheit ausführbare Steuerinformationen speichert, und wobei die MR-Anlage ausgebildet ist, bei der Ausführung der Steuerinformationen in der MR-Steuereinheit die folgenden Schritte auszuführen. In einem Schritt wird ein Magnetfeld an ein Bildgebungsvolumen und an ein Zuflussvolumen, von dem Flüssigkeit in das Bildgebungsvolumen eintritt, einer Untersuchungsperson angelegt. In einem weiteren Schritt wird das Bildgebungsvolumen durch einen HF-Puls, welcher eine Magnetisierungs-Transferfunktion und eine Fett-Sättigungsfunktion erfüllt, angeregt während das Magnetfeld angelegt ist. In einem zusätzlichen Schritt werden MR-Signale aus dem Bildgebungsvolumen zum Darstellen von Gefäßen gemessen. Dabei weist der HF-Puls eine Frequenzverteilung auf, deren Frequenzen im Wesentlichen auf der Seite der Fettfrequenz liegen, und welche die Fettfrequenz im Bildgebungsvolumen umfassen. Weiter weist das Magnetfeld eine Magnetfeldverteilung auf, welche durch eine Funktion angenähert ist, die derart ausgebildet ist, dass sie einen Scheitelbereich mit im Wesentlichen keinem räumlichen Gradienten im Bildgebungsvolumen aufweist und einen höheren räumlichen Gradienten im Zuflussvolumen aufweist, so dass die Wasserfrequenz im Zuflussvolumen nicht von dem HF-Puls umfasst wird.

Die MR-Anlage zur Gefäßdarstellung kann derart ausgebildet sein, dass sie der Ausführung der Steuerinformationen in der MR-Steuereinheit das Verfahren entsprechend der weiteren oben beschriebenen Merkmale auszuführen.

Für eine derartige MR-Anlage zur Gefäßdarstellung können technische Effekte erzielt werden, die vergleichbar sind mit den technischen Effekten, die für das Verfahren gemäß dem ersten Aspekt obenstehend beschrieben wurden.

Gemäß einem weiteren Aspekt der Erfindung wird ein Computerprogrammprodukt bereitgestellt, welches ein Programm umfasst, das direkt in einen Speicher einer MR-Steuereinheit einer MR-Anlage ladbar ist, mit Programm-Mitteln, um die Schritte des Verfahrens entsprechend den unter dem ersten Aspekt der Erfindung beschriebenen Merkmale auszuführen, wenn das Programm in der MR-Steuereinheit der MR-Anlage ausgeführt wird.

Gemäß einem weiteren Aspekt der Erfindung wird ein elektronisch lesbarer Datenträger mit darauf gespeicherten elektronisch lesbaren Steuerinformationen bereitgestellt, welche derart ausgestaltet sind, dass sie bei Verwendung des Datenträgers in einer MR-Steuereinheit einer MR-Anlage das Verfahren entsprechend den unter dem ersten Aspekt der Erfindung beschriebenen Merkmalen durchführen.

Die oben dargelegten Merkmale und Merkmale, die nachfolgend beschrieben werden, können nicht nur in den entsprechenden explizit dargelegten Kombinationen verwendet werden, sondern auch in weiteren Kombinationen oder isoliert, ohne den Schutzumfang der vorliegenden Erfindung zu verlassen.

### Kurze Beschreibung der Zeichnungen

Die Erfindung wird nachfolgend unter Bezugnahme auf die beiliegenden Zeichnungen näher erläutert.
Figur 1 zeigt schematisch eine MR-Anlage, mit der ein Verfahren zur Gefäßdarstellung erfindungsgemäß durchgeführt werden kann.
Figur 2 zeigt eine schematische Darstellung einer Untersuchungsperson mit zugehörigen Frequenzverläufen ohne erfindungsgemäß angelegtes Magnetfeld.
Figur 3 zeigt eine schematische Darstellung der Untersuchungsperson der Figur 2 mit zugehörigen Frequenzverläufen bei angelegtem Magnetfeld der Erfindung.
Figuren 4a-c zeigen einen ersten HF-Puls zur Erzeugung des HF-Pulses gemäß einem Ausführungsbeispiel gemäß einem Ausführungsbeispiel der Erfindung.
Figuren 5a-c zeigen einen zweiten HF-Puls zur Erzeugung des HF-Pulses gemäß einem Ausführungsbeispiel der Erfindung.
Figur 6 zeigt einen HF-Puls gemäß einem Ausführungsbeispiel der Erfindung.
Figur 7 zeigt ein Flussdiagramm mit Schritten zur Durchführung eines Verfahrens zur Gefäßdarstellung gemäß einem Ausführungsbeispiel der Erfindung.

### Detaillierte Beschreibung

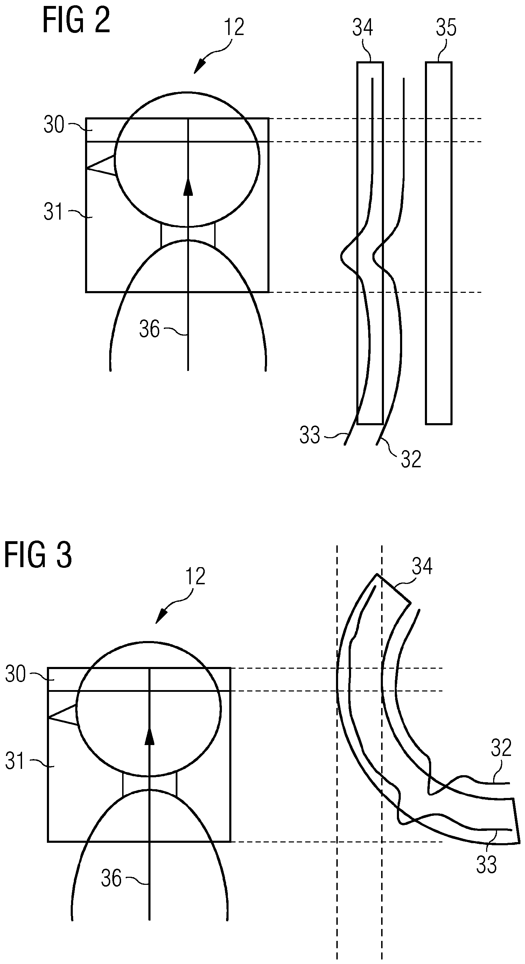

Die vorliegende Erfindung betrifft ein Verfahren zur Gefäßdarstellung mit Hilfe einer MR-Anlage. Bezugnehmend auf Fig. 2 wird bei einer Gefäßdarstellung basierend auf der TOF-Technik eine ortsfeste Magnetisierung insbesondere von Gehirngewebe, Spinalflüssigkeit und Fett, welche ein unerwünschtes Hintergrundsignal in einem Bildgebungsvolumen 30 erzeugen, durch einen HF-Puls 40 bei gleichzeitig angelegtem erfindungsgemäßem Magnetfeld gesättigt. Dabei sind der HF-Puls 40 und das angelegte Magnetfeld derart ausgebildet, dass sie die Hintergrundsignale unterdrücken, ohne die in das Bildgebungsvolumen zufließenden Spins der Gefäßflüssigkeit anzuregen.

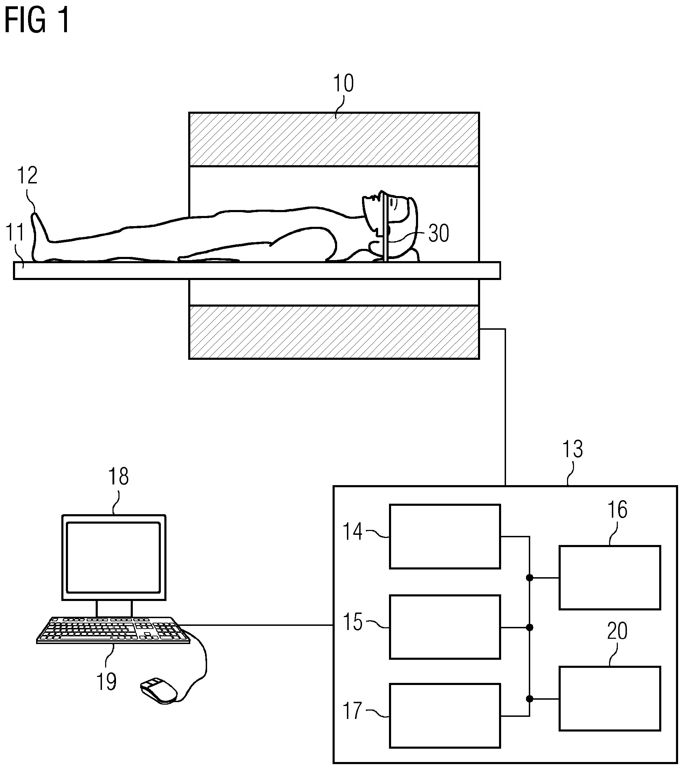

Figur 1 zeigt schematisch eine MR-Anlage, mit der ein Verfahren zur Gefäßdarstellung erfindungsgemäß durchgeführt werden kann.

Eine Untersuchungsperson 12, oder allgemeiner ein Untersuchungsobjekt, ist in den Tunnel der Anlage gefahren. Die Magnetresonanzanlage weist einen Magneten 10 zur Erzeugung eines Grundfeldes B0 auf, wobei eine auf einer Liege 11 angeordnete Untersuchungsperson 12 in das Zentrum des Magneten gefahren wird, um dort ortscodierte Magnetresonanzsignale aus einem Untersuchungsabschnitt aufzunehmen. Durch Einstrahlen von Hochfrequenzpulsfolgen und Schalten von Magnetfeldgradienten kann die durch das Grundfeld B0 erzeugte Magnetisierung gestört werden durch Auslenkung der Kernspins aus der Gleichgewichtslage, und die bei der Rückkehr in die Gleichgewichtslage in Empfangsspulen induzierten Ströme können in Magnetresonanzsignale umgewandelt werden. Die allgemeine Funktionsweise zur Erstellung von MR-Bildern und die Detektion der Magnetresonanzsignale sind dem Fachmann bekannt, sodass auf eine detaillierte Erläuterung hiervon verzichtet wird.

Die Magnetresonanzanlage weist weiterhin eine MR-Steuereinheit 13 auf, die zur Steuerung des MR-Geräts verwendet wird. Die zentrale MR-Steuereinheit 13, welche derart ausgebildet ist, dass die das unten beschriebene Verfahren für eine automatische Justierung durchführt, weist eine Gradientensteuerung 14 zur Steuerung und Schaltung der Magnetfeldgradienten auf und eine HF-Steuerung 15 zur Steuerung und Einstrahlung der HF-Pulse zur Auslenkung der Kernspins aus der Gleichgewichtlage. In einer Speichereinheit 16 können beispielsweise die für die Aufnahme der MR-Bilder notwendigen Bildgebungssequenzen abgespeichert werden, sowie alle Programme, die zum Betrieb der MR-Anlage notwendig sind. Eine Aufnahmeeinheit 17 steuert die Bildaufnahme und steuert damit in Abhängigkeit von den gewählten Bildgebungssequenzen die Abfolge der Magnetfeldgradienten und HF-Pulse und die Empfangsintervalle von MR-Signalen. Somit steuert die Aufnahmeeinheit 17 auch die Gradientensteuerung 14 und die HF-Steuerung 15. In einer Recheneinheit 20 können MR-Bilder berechnet werden, die auf eine Anzeige 18 angezeigt werden können, wobei eine Bedienperson über eine Eingabeeinheit 19 die MR-Anlage bedienen kann. Die Speichereinheit 16 kann Bildgebungssequenzen und Programmmodule aufweisen, die bei Ausführung in der Recheneinheit 20 von einem der gezeigten Module, das erfindungsgemäße Verfahren durchführen. Die HF-Steuerung 15 kann weiterhin ausgebildet sein, eine Unterdrückung von Hintergrundsignalen bei einer Gefäßdarstellung basierend auf der Time-of-Flight (TOF)-Technik zu verbessern, wie nachfolgend im Detail erläutert wird. Insbesondere speichert die Speichereinheit 16 dazu von der MR-Steuereinheit 13 ausführbare Steuerinformationen. Weiter ist die Aufnahmeeinheit 17 derart ausgebildet, dass sie das nachfolgend beschriebene Verfahren zur Gefäßdarstellung durchführen kann.

Erfindungsgemäß ist die MR-Anlage der Figur 1 derart ausgebildet, dass sie bei der Ausführung der Steuerinformationen in der MR-Steuereinheit 13 ein Magnetfeld an ein Bildgebungsvolumen 30 und an ein Zuflussvolumen 31, von dem Flüssigkeit in das Bildgebungsvolumen 30 eintritt, einer Untersuchungsperson 12 anlegt wie in Fig. 2 gezeigt. Weiter wird das Bildgebungsvolumen 30 durch einen HF-Puls 40 anregt, welcher eine Magnetisierungs-Transferfunktion und eine Fett-Sättigungsfunktion erfüllt, während das Magnetfeld angelegt ist. Weiterhin werden MR-Signale aus dem Bildgebungsvolumen 30 zum Darstellen von Gefäßen, welche sich in dem Bildgebungsvolumen befinden, gemessen. Dabei weist der HF-Puls 40 eine Frequenzverteilung auf, deren Frequenzen im Wesentlichen niedriger als die Mittenfrequenz des Wassers 32 im Bildgebungsvolumen 30 sind, und welche die Fettfrequenz 33 im Bildgebungsvolumen 30 umfasst. Zudem ist eine Feldverteilung des Magnetfelds durch eine Funktion angenähert, die derart ausgebildet ist, dass sie einen Scheitelbereich mit im Wesentlichen keinem räumlichen Gradienten im Bildgebungsvolumen 30 aufweist und einen höheren räumlichen Gradienten im Zuflussvolumen 31 aufweist.

Figur 2 zeigt eine schematische Darstellung einer Untersuchungsperson mit zugehörigen Frequenzverläufen der Wasserfrequenz und Fettfrequenz ohne erfindungsgemäß angelegtes Magnetfeld.

Eine Untersuchungsperson 12 weist ein Bildgebungsvolumen 30 und ein Zuflussvolumen 31 auf. Ein Blutgefäß 36 ist in dem Bildgebungsvolumen 30 und dem Zuflussvolumen 31 enthalten, sodass aus dem Zuflussvolumen 31 Blut in das Bildgebungsvolumen hineinfließt.

Das Bildgebungsvolumen 30 und das Zuflussvolumen 31 sind in diesem Ausführungsbeispiel schichtförmig ausgebildet. Zudem ist eine Längsachse senkrecht zu dem Bildgebungsvolumen 30 und dem Zuflussvolumen 31 ausgerichtet, und wobei entlang der Längsachse eine Schichtdicke des Zuflussvolumens (31) gleich einem Vielfachen einer Schichtdicke des Bildgebungsvolumens (30) ist. Das Blutgefäß 36 verläuft ebenfalls im Wesentlichen entlang der Längsachse.

Die Untersuchungsperson 12 weist unter dem Einfluss des B0-Feldes eine Wasserfrequenz 32 auf, welche der Flüssigkeit in dem Gefäß, insbesondere dem Blut in dem Blutgefäß, entspricht. Die Wasserfrequenz 32 weist einen räumlichen Verlauf in z-Richtung, oder Kopf-Fuß-Richtung der Untersuchungsperson 12, auf. Im Bereich des Bildgebungsvolumens 30 verläuft die Wasserfrequenz 32 konstant, wogegen die Wasserfrequenz aufgrund von Inhomogenitäten des Gradientenfeldes innerhalb des Zuflussvolumens 31 in einem Teilbereich des Zuflussvolumens 31 verschoben ist. In diesem Teilbereich verläuft die Wasserfrequenz 32 in dem Fett-Sättigungs-Band 34. Weiter weist die Untersuchungsperson 12 unter dem Einfluss des B0 Feldes eine Fettfrequenz 33 auf, welche Fettansammlungen, Fettanlagerungen oder anderem fetthaltigem Gewebe der Untersuchungsperson 12 im Bildgebungsvolumen 30 entspricht. Im Bereich des Bildgebungsvolumens 30 verläuft die Fettfrequenz 33 innerhalb eines Fett-Sättigungs-Bandes 34. Innerhalb des Zuflussvolumens 31 weist die Fettfrequenz 33 eine entsprechende Frequenzverschiebung auf wie die Wasserfrequenz 32. Ein Magnetisierungs-Transfer-Band 35 verläuft neben der Wasserfrequenz 32 auf der Seite, welche der Fettfrequenz abgewandt ist, oder in anderen Worten neben der Wasserfrequenz 32 in Richtung höherer Frequenzen, ohne Überschneidungen mit der Wasserfrequenz 32. In den Randbereichen der Untersuchungsperson fällt das B0-Feld ab, sodass auch die Wasserfrequenz 32 und die Fettfrequenz 32 abfallen. Dies bedeutet, dass bei Verwendung eines Sättigungs-Pulses mit dem Band 34 Teile des einfließenden Blutes mit gesättigt werden würden, was unerwünscht ist.

Figur 3 zeigt eine schematische Darstellung der Untersuchungsperson der Figur 2 mit mit zugehörigen Frequenzverläufen bei angelegtem Magnetfeld gemäß einem Ausführungsbeispiel der Erfindung.

Während das Magnetfeld angelegt ist, sind die Resonanzfrequenzen im Einflussbereich des Magnetfelds, und insbesondere die Resonanzfrequenzen im Zuflussvolumen 31, verschoben. Erfindungsgemäß ist das Magnetfeld derart ausgebildet, dass die Verschiebung der Resonanzfrequenzen im Zuflussvolumen 31 mit einem im Vergleich zum Bildgebungsvolumen 30 höheren Gradienten, insbesondere nichtlinear mit einem Abstand in z-Richtung zu dem Bildgebungsvolumen 30, ansteigt.

Insbesondere ist die Wasserfrequenz 32 im Zuflussvolumen 31 derart in Richtung von der dem Fett-Sättigungs-Band 34 weg verschoben, dass ein nichtselektiv eingestrahlter Fett-Sättigungs-Puls, welcher eine Frequenzverteilung aufweist, die dem Fett-Sättigungs-Band 34 im Bereich des Bildgebungsvolumens 30 entspricht, die Wasserfrequenz 32 im Bereich des Zuflussvolumens 31 nicht umfasst. Dadurch wird Blut, welches sich im Blutgefäß 36 im Zuflussvolumen 31 befindet und welches in das Bildgebungsvolumen 30 fließt, nicht angeregt.

In einem Ausführungsbeispiel, in welchem das Blutgefäß 36 in der Nähe des Isozentrums des Bildgebungsvolumens 30 gelegen ist, kann das Anlegen des Magnetfeldes durch eine Anpassung des z2-Shims erfolgen, wobei in Folge auch die übrigen Shims zweiter Ordnung angepasst werden müssen.

In einem anderen Ausführungsbeispiel kann ein einfacher Shim-Algorithmus aus folgenden Schritten bestehen: Optimieren der B0-Homogenität in dem Bildgebungsvolumen 30 mit der Randbedingung, den z2-Shim bei einem fixen Wert zu halten, z.B. bei dem Tune-Up-Wert plus 1500 µT/M^2.
In einem Ausführungsbeispiel werden die Schritte des Verfahrens für mindestens ein weiteres Bildgebungsvolumen und mindestens ein weiteres Zuflussvolumen durchgeführt. Bedingung für eine Fett-Sättigung über das gesamte Bildgebungsvolumen limitiert den Wert des z2-Offsets. Dieses Limit skaliert mit einem Erstrecken des Bildgebungsvolumens 30 in z-Richtung. Die TOF-Technik wird immer unter Verwenden der Multiple-Overlapping-Transverse-Slab-Acquisition (MOTSA)-Technik durchgeführt. Zum Beispiel wird ein transverses Untersuchungsvolumen mit 10 cm Schichtdicke durch 6 überlappende Bildgebungsvolumina 30 mit 2 cm Schichtdicke abgedeckt. Dabei werden die Bildgebungsvolumina 30 sequentiell gemessen, daher kann das Magnetfeld für jedes Bildgebungsvolumen 30 einzeln optimiert und angepasst werden. In einem Ausführungsbeispiel wird die quadratische Feldverteilung entlang der z-Achse verschoben. Bei schrägen Bildgebungsvolumina (typischerweise T>C) muss der z2-Wert reduziert werden, damit er mit der Dicke des Bildgebungsvolumens übereinstimmt, wenn diese auf die z-Achse projiziert wird. In einem anderen Ausführungsbeispiel muss die Parabelrichtung unter Verwenden der anderen Shim-Kanäle zweiter Ordnung geneigt werden.

Somit wird die Magnetfeldverteilung an das mindestens eine weiteren Bildgebungsvolumen und das mindestens eine weitere Zuflussvolumen derart angepasst, dass die angepasste Magnetfeldverteilung in dem mindestens einen weiteren Bildgebungsvolumen den Scheitelbereich aufweist und in dem mindestens einen weiteren Zuflussvolumen den höheren räumlichen Gradienten aufweist.

In einem Ausführungsbeispiel werden mehrere Messungen pro HF-Puls 40 durchgeführt. Die HF-Pulse 40 sollten die Bildaufnahmedauer nicht verlängern. Daher werden pro HF-Puls mehrere Auslesungen durchgeführt, wobei der durchschnittliche zeitliche Abstand zwischen den Auslesungen konstant gehalten wird, um den TOF-Kontrast beizubehalten, z.B. können statt einer Auslesung pro Repetitionszeit TR = 24 ms auch zwei Auslesungen in 48 ms oder drei Auslesungen in 72 ms durchgeführt werden. Die Bildqualität ist unabhängig von der Positionierung der Auslesungen in der TR, sie können äquidistant oder ohne Pause ausgespielt werden, wodurch eine größere Pause pro TR gelassen wird, welche groß genug ist, um einen langen MT- und Fett-Sättigungs-Puls auszuspielen, welcher ein wohldefiniertes Frequenzprofil und ein niedriges SAR aufweist.

In einem Ausführungsbeispiel werden TSAT-Pulse und MT-Pulse abwechselnd ausgespielt. In einem weiteren Ausführungsbeispiel werden TSAT-Pulse und MT-Pulse abwechselnd mit variierender Repetitionszeit TR ausgespielt. In einem weiteren Ausführungsbeispiel werden mindestens ein TSAT-Puls und mindestens ein MT-Puls in einer Repetitionszeit TR ausgespielt.

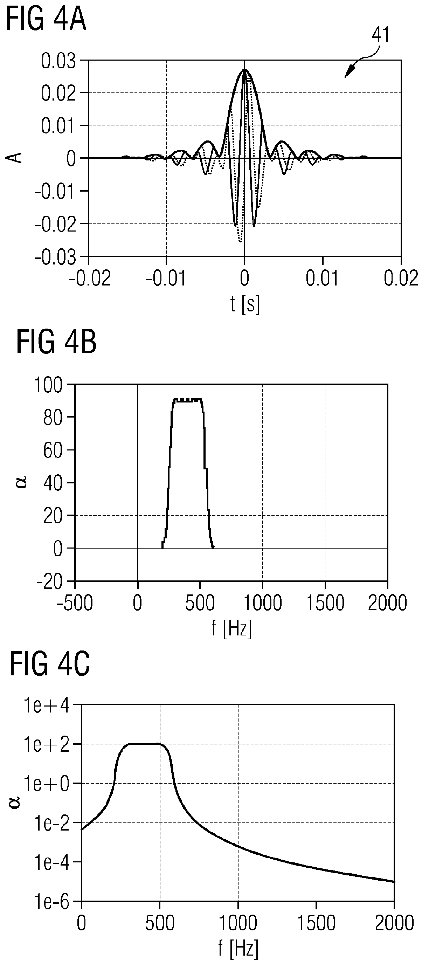

Figuren 4a-c zeigen einen ersten HF-Puls zur Erzeugung des HF-Pulses gemäß einem Ausführungsbeispiel gemäß einem Ausführungsbeispiel der Erfindung.

Figur 4a zeigt eine Darstellung einer komplexen Amplitude A eines ersten HF-Pulses 41 über Zeit.

Figur 4b zeigt den Kippwinkel α des ersten HF-Pulses 41 im Frequenzraum.

Figur 4c zeigt den Kippwinkel α des ersten HF-Pulses 41 im Frequenzraum in logarithmischer Darstellung.

Der erste HF-Puls 41 weist vorzugsweise einen Flip-Winkel von 20 bis 90 Grad, besonders vorzugsweise 90 Grad, und eine Frequenz im Wesentlichen gleich der Fettfrequenz 33 im Bildgebungsvolumen 30 auf.

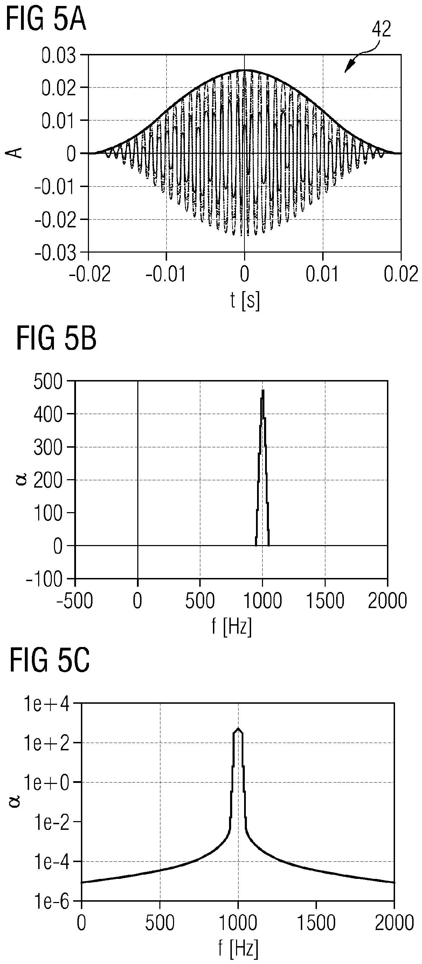

Figuren 5a-c zeigen einen zweiten HF-Puls zur Erzeugung des HF-Pulses gemäß einem Ausführungsbeispiel der Erfindung.

Figur 5a zeigt eine Darstellung einer komplexen Amplitude A eines zweiten HF-Pulses 42 über Zeit.

Figur 5b zeigt den Kippwinkel α des zweiten HF-Pulses 42 im Frequenzraum.

Figur 5c zeigt den Kippwinkel α des zweiten HF-Pulses 42 im Frequenzraum in logarithmischer Darstellung.

Der zweite HF-Puls 42 weist vorzugsweise einen Flip-Winkel von einigen hundert Grad, besonders vorzugsweise 500 bis 600 Grad, und eine Frequenz von 1 kHz bis 1,5 kHz, besonders vorzugsweise 1,5 kHz, auf.

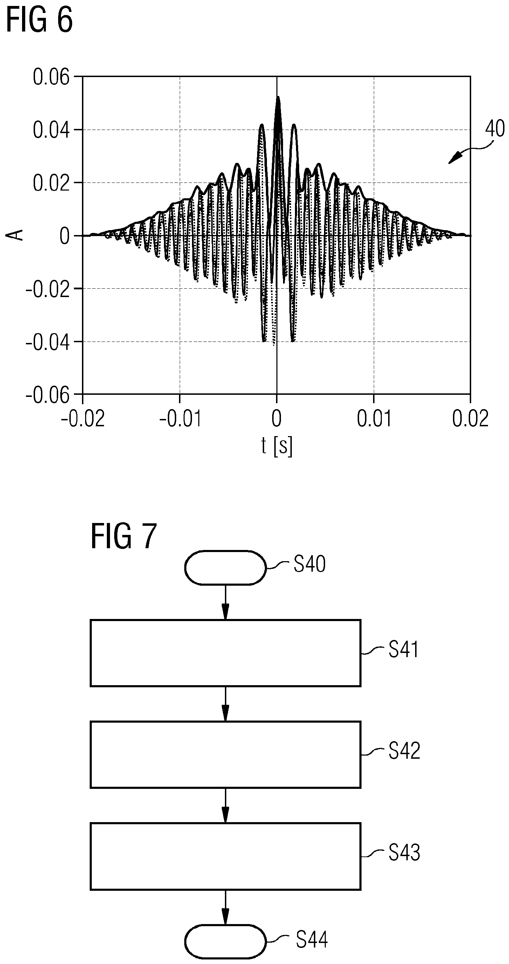

Figur 6 zeigt einen HF-Puls 40 gemäß einem Ausführungsbeispiel der Erfindung.

Der HF-Puls 40 wurde erzeugt durch Überlagerung des ersten HF-Pulses 41, wie in den Figuren 4 a-c gezeigt, und des zweiten HF-Pulses 42, wie in den Figuren 5 a-c gezeigt.

In einem Ausführungsbeispiel wird der HF-Puls für Fett-Sättigung und MT optimiert. MT benötigt einen viel höheren Kippwinkel als eine Fett-Sättigung. Für eine effektive Fett-Sättigung genügt bereits ein Kippwinkel von nicht mehr als 90 Grad pro TR (50 ms), während der MT-Kippwinkel so hoch wie möglich sein sollte. In einigen Ausführungsbeispielen ist der MT-Kippwinkel 6-10 mal so hoch wie der Kippwinkel für die Fett-Sättigung. Unerwünschte Sättigung der Wasserfrequenz erhöht sich mit dem Kippwinkel und verringert sich mit zunehmendem Abstand von der Wasserfrequenz und SAR intensiven Pulse-Profil-Optimierungen. Da der MT-Effekt nur in geringem Maße vom Abstand von der Wasserfrequenz abhängt, kann der HF-Puls derart optimiert werden, dass er den Kippwinkel, welcher für die Fett-Sättigung notwendig ist, bereitstellt und den übrigen Kippwinkel bei einem größeren Frequenzabstand neben der Wasserfrequenz bereitstellt, z.B. bei 1,5 kHz. Dies wird durch das Überlagern der Pulshüllen des ersten HF-Pulses 41 und des zweiten HF-Pulses 42 erreicht, z.B. HF-Pulse mit 2ex20: 90 Grad bei der Fett-Sättigungs-Frequenz und 500 Grad bei 1 kHz.

In einem Ausführungsbeispiel weist der HF-Puls 40 vorzugsweise einen uniformen Kippwinkel von 90 Grad bei ungefähr 400 Hz neben der Wasserfrequenz 32, und einen Kippwinkel von 500 Grad in der Nähe von 1 kHz neben der Wasserfrequenz 32 auf, wobei der Kippwinkel bei der Wasserfrequenz vernachlässigbar, oder kleiner 1 Grad, ist. Der HF-Puls 40 befindet sich auf der Seite der Wasserfrequenz 32, auf der die Fettfrequenz 33 liegt.

Figur 7 zeigt ein Flussdiagramm mit Schritten zur Durchführung eines Verfahrens zur Gefäßdarstellung gemäß einem erfindungsgemäßen Ausführungsbeispiel.

Das Verfahren beginnt in Schritt S40. In Schritt S41 wird ein Magnetfeld an ein Bildgebungsvolumen 30 und ein Zuflussvolumen 31 eine Untersuchungsperson 12 angelegt. In Schritt S42 wird das Bildgebungsvolumen 30 durch einen HF-Puls 40, welcher eine Magnetisierungs-Transferfunktion eine Fett-Sättigungsfunktion erfüllt, angeregt während das Magnetfeld angelegt angelegt ist. Dabei weist der HF-Puls 40 eine Frequenzverteilung auf, deren Frequenzen im Wesentlichen niedriger als die Mittenfrequenz des Wassers 32 im Bildgebungsvolumen 30 sind, und welche die Fettfrequenz 33 im Bildgebungsvolumen 30 umfassen. Das Magnetfeld weist eine Magnetfeldverteilung auf, welche einen Scheitelbereich mit im Wesentlichen keinem räumlichen Gradienten im Bildgebungsvolumen 30 aufweist und einen höheren räumlichen Gradienten im Zuflussvolumen 31 aufweist. In Schritt S43 werden MR-Signale aus dem Bildgebungsvolumen 30 zum Darstellen von Gefäßen gemessen. Das Verfahren endet in Schritt S44.

Zusammenfassend wird ein Verfahren zur Gefäßdarstellung mit Hilfe einer Magnetresonanz-Anlage basierend auf der TOF-Technik bereitbestellt, wobei eine ortsfeste Magnetisierung insbesondere von von Gehirngewebe, Spinalflüssigkeit und Fett in einem Bildgebungsvolumen 30, welche unerwünschte Hintergrundsignale erzeugen, erfindungsgemäß durch einen HF-Puls 40 bei gleichzeitig angelegtem Magnetfeld unterdrückt. Erfindungsgemäß werden bei dem Verfahren zur Gefäßdarstellung Hintergrundsignale unterdrückt, ohne die in das Bildgebungsvolumen zufließenden Spins der Gefäßflüssigkeit zu beeinflussen. Dadurch wird eine schnellere und effizientere MR-Bildgebung zur Gefäßdarstellung ermöglicht, welche durch einen geringen Zeit- und Personalaufwand verringerte Untersuchungskosten aufweist.

## Patentansprüche

1. Verfahren zur Gefäßdarstellung basierend auf der Time-of-Flight (TOF)-Technik mit Hilfe einer MR-Anlage, umfassend folgende Schritte:
- Anlegen eines Magnetfeldes an ein Bildgebungsvolumen (30) und an ein Zuflussvolumen (31), von dem Flüssigkeit in das Bildgebungsvolumen (30) eintritt, einer Untersuchungsperson (12, S41);
- Anregen des Bildgebungsvolumens (30) durch einen HF-Puls (40), welcher eine Magnetisierungs-Transferfunktion und eine Fett-Sättigungsfunktion erfüllt, während das Magnetfeld angelegt ist (S42); und
- Messen von MR-Signalen aus dem Bildgebungsvolumen (30) zum Darstellen von Gefäßen (S43);
**dadurch gekennzeichnet, dass** der HF-Puls (40) eine Frequenzverteilung aufweist, deren Frequenzen im Wesentlichen auf der Seite der Fettfrequenz liegen, und welche die Fettfrequenz (33) im Bildgebungsvolumen (30) umfassen; und
dass das Magnetfeld eine Magnetfeldverteilung aufweist, welche durch eine Funktion angenähert ist, die derart ausgebildet ist, dass sie einen Scheitelbereich mit im Wesentlichen keinem räumlichen Gradienten im Bildgebungsvolumen (30) aufweist und einen höheren räumlichen Gradienten im Zuflussvolumen (31) aufweist, so dass die Wasserfrequenz im Zuflussvolumen (31) nicht von dem HF-Puls umfasst wird.

2. Verfahren nach Anspruch 1, wobei die Funktion eine mathematische Funktion mindestens zweiter Ordnung ist.

3. Verfahren nach Anspruch 1 oder 2, wobei das Bildgebungsvolumen (30) und das Zuflussvolumen (31) schichtförmig sind, wobei eine Längsachse senkrecht zu dem Bildgebungsvolumen (30) und dem Zuflussvolumen (31) ausgerichtet ist, und wobei entlang der Längsachse eine Schichtdicke des Zuflussvolumens (31) gleich einem Vielfachen einer Schichtdicke des Bildgebungsvolumens (30) ist.

4. Verfahren nach einem der Ansprüche 1 bis 3, wobei der Scheitelbereich der Funktion im Wesentlichen die gleiche Dicke hat, wie die Schichtdicke des Bildgebungsvolumens (30) .

5. Verfahren nach einem der vorhergehenden Ansprüche, wobei die Schritte des Verfahrens für mindestens ein weiteres Bildgebungsvolumen und mindestens ein weiteres Zuflussvolumen durchgeführt werden, weiter umfassend:
- Anpassen der Magnetfeldverteilung an das mindestens eine weiteren Bildgebungsvolumen und das mindestens eine weitere Zuflussvolumen, derart, dass die angepasste Magnetfeldverteilung in dem mindestens einen weiteren Bildgebungsvolumen den Scheitelbereich aufweist und in dem mindestens einen weiteren Zuflussvolumen den höheren räumlichen Gradienten aufweist.

6. Verfahren nach Anspruch 5, wobei das Anpassen der Magnetfeldverteilung ein lineares räumliches Verschieben der Magnetfeldverteilung umfasst.

7. Verfahren nach Anspruch 5 oder 6, wobei das Anpassen der Magnetfeldverteilung ein Ändern einer linearen Komponente der Magnetfeldverteilung umfasst.

8. Verfahren nach einem der Ansprüche 5 bis 7, wobei die Bildgebungsvolumina sich zumindest teilweise räumlich überschneiden.

9. Verfahren nach einem der vorhergehenden Ansprüche, weiter umfassend:
- Erzeugen des HF-Pulses (40) durch Überlagern eines ersten HF-Pulses (41), welcher eine Fett-Sättigungsfunktion erfüllt, und eines zweiten HF-Pulses (42), welcher eine Magnetisierungs-Transferfunktion erfüllt.

10. Verfahren nach Anspruch 9, wobei der erste HF-Puls (41) einen ersten Flip-Winkel und der zweite HF-Puls (42) einen zweiten Flip-Winkel unterschiedlich zu dem ersten Flip-Winkel aufweist.

11. Verfahren nach Anspruch 9 oder 10, wobei der erste HF-Puls (41) vorzugsweise einen Flip-Winkel von 20 bis 90 Grad, besonders vorzugsweise 90 Grad, und eine Frequenz im Wesentlichen gleich der Fettfrequenz (33) im Bildgebungsvolumen (30) aufweist.

12. Verfahren nach einem der vorhergehenden Ansprüche, wobei pro HF-Puls (40) mehrere Messungen von MR-Signalen aus dem Bildgebungsvolumen (30) durchgeführt werden.

13. Verfahren nach Anspruch 12, wobei die Messungen in gleichen zeitlichen Abständen durchgeführt werden.

14. Verfahren nach Anspruch 12, wobei die Messungen in Messgruppierungen mit zeitlichen Abständen zwischen den einzelnen Messungen durchgeführt werden, wobei zwischen den Messgruppierungen eine Anregung durch einen HF-Puls (40) durchgeführt wird, und wobei der mittlere zeitliche Abstand zwischen den Messungen kleiner einem vorbestimmten Schwellenwert ist.

15. MR-Anlage zur Gefäßdarstellung basierend auf der Time-of-Flight (TOF)-Technik, wobei die MR-Anlage eine MR-Steuereinheit (13) und eine Speichereinheit (16) aufweist, wobei die Speichereinheit (16) von der MR-Steuereinheit (13) ausführbare Steuerinformationen speichert, und wobei die MR-Anlage ausgebildet ist, bei der Ausführung der Steuerinformationen in der MR-Steuereinheit (13) folgende Schritte auszuführen:
- Anlegen eines Magnetfeldes an ein Bildgebungsvolumen (30) und an ein Zuflussvolumen (31), von dem Flüssigkeit in das Bildgebungsvolumen (30) eintritt, einer Untersuchungsperson (12);
- Anregen des Bildgebungsvolumens (30) durch einen HF-Puls (40), welcher eine Magnetisierungs-Transferfunktion und eine Fett-Sättigungsfunktion erfüllt, während das Magnetfeld angelegt ist; und
- Messen von MR-Signalen aus dem Bildgebungsvolumen (30) zum Darstellen von Gefäßen;
**dadurch gekennzeichnet, dass** der HF-Puls (40) eine Frequenzverteilung aufweist, deren Frequenzen im Wesentlichen auf der Seite der Fettfrequenz liegen, und welche die Fettfrequenz (33) im Bildgebungsvolumen (30) umfasst; und
dass eine Feldverteilung des Magnetfelds durch eine Funktion angenähert ist, die derart ausgebildet ist, dass sie einen Scheitelbereich mit im Wesentlichen keinem räumlichen Gradienten im Bildgebungsvolumen (30) aufweist und einen höheren räumlichen Gradienten im Zuflussvolumen (31) aufweist, so dass die Wasserfrequenz im Zuflussvolumen (31) nicht von dem HF-Puls umfasst wird.

16. MR-Anlage zur Gefäßdarstellung, wobei die MR-Anlage eine MR-Steuereinheit (13) und eine Speichereinheit (16) aufweist, wobei die Speichereinheit (16) von der MR-Steuereinheit (13) ausführbare Steuerinformationen speichert, und wobei die MR-Anlage ausgebildet ist, bei der Ausführung der Steuerinformationen in der MR-Steuereinheit (13) das Verfahren eines der Ansprüche 2 bis 14 auszuführen.

17. Computerprogrammprodukt, welches ein Programm umfasst, das direkt in einen Speicher einer MR-Steuereinheit (13) einer MR-Anlage ladbar ist, mit Programm-Mitteln, um die Schritte des Verfahrens nach einem der Ansprüche 1 bis 14 auszuführen, wenn das Programm in der MR-Steuereinheit (13) der MR-Anlage ausgeführt wird.

18. Elektronisch lesbarer Datenträger mit darauf gespeicherten elektronisch lesbaren Steuerinformationen, welche derart ausgestaltet sind, dass sie bei Verwendung des Datenträgers in einer MR-Steuereinheit (13) einer MR-Anlage das Verfahren nach einem der Ansprüche 1 bis 14 durchführen.

## Claims

1. Method of vascular imaging based on the Time-of-Flight (TOF) technique with the aid of an MR system, comprising the following steps:
- applying a magnetic field to an imaging volume (30) and to an inflow volume (31), from which liquid enters into the imaging volume (30), of an examination person (12, S41) ;
- exciting the imaging volume (30) by way of an RF pulse (40), which fulfils a magnetisation transfer function and a fat saturation function, while the magnetic field is being applied (S42); and
- measuring MR signals from the imaging volume (30) in order to visualise vessels (S43);
**characterised in that** the RF pulse (40) has a frequency distribution whose frequencies are essentially located on the side of the fat frequency, and which comprise the fat frequency (33) in the imaging volume (30); and
the magnetic field has a magnetic field distribution, which is approximated by a function that is configured in such a way that it has an apex with essentially no spatial gradient in the imaging volume (30) and has a higher spatial gradient in the inflow volume (31), so that the water frequency in the inflow volume (31) is not comprised by the RF pulse.

2. Method according to claim 1, wherein the function is a mathematical function of at least the second order.

3. Method according to claim 1 or 2, wherein the imaging volume (30) and the inflow volume (31) are slice-like, wherein a longitudinal axis is oriented perpendicularly to the imaging volume (30) and the inflow volume (31), and wherein along the longitudinal axis, a slice thickness of the inflow volume (31) is equal to a multiple of a slice thickness of the imaging volume (30).

4. Method according to one of claims 1 to 3, wherein the apex of the function has essentially the same thickness as the slice thickness of the imaging volume (30).

5. Method according to one of the preceding claims, wherein the steps of the method are carried out for at least one further imaging volume and at least one further inflow volume, also comprising:
- adjusting the magnetic field distribution to the at least one further imaging volume and the at least one further inflow volume, in such a way that the adjusted magnetic field distribution has the apex in the at least one further imaging volume and the higher spatial gradient in the at least one further inflow volume.

6. Method according to claim 5, wherein the adjustment of the magnetic field distribution comprises a linear spatial shift of the magnetic field distribution.

7. Method according to claim 5 or 6, wherein the adjustment of the magnetic field distribution comprises a change in a linear component of the magnetic field distribution.

8. Method according to one of claims 5 to 7, wherein the imaging volumes at least partially spatially overlap.

9. Method according to one of the preceding claims, also comprising:
- generating the RF pulse (40) by overlaying a first RF pulse (41), which fulfils a fat saturation function, and a second RF pulse (42), which fulfils a magnetisation transfer function.

10. Method according to claim 9, wherein the first RF pulse (41) has a first flip angle and the second RF pulse (42) has a second flip angle different from the first flip angle.

11. Method according to claim 9 or 10, wherein the first RF pulse (41) preferably has a flip angle of 20 to 90 degrees, particularly preferably 90 degrees, and a frequency essentially equal to the fat frequency (33) in the imaging volume (30).

12. Method according to one of the preceding claims, wherein a plurality of measurements of MR signals from the imaging volume (30) are carried out per RF pulse (40).

13. Method according to claim 12, wherein the measurements are carried out at regular time intervals.

14. Method according to claim 12, wherein the measurements are carried out in measurement groups with time intervals between the individual measurements, wherein an excitation is carried out by an RF pulse (40) between the measurement groups, and wherein the mean time interval between the measurements is shorter than a predetermined threshold value.

15. MR system for vascular imaging based on the Time-of-Flight (TOF) technique, wherein the MR system has an MR control unit (13) and a storage unit (16), wherein the storage unit (16) stores control information that can be executed by the MR control unit (13), and wherein the MR system is designed to carry out the following steps when the control information is executed in the MR control unit (13):
- applying a magnetic field to an imaging volume (30) and to an inflow volume (31), from which liquid enters into the imaging volume (30), of an examination person (12);
- exciting the imaging volume (30) by way of an RF pulse (40), which fulfils a magnetisation transfer function and a fat saturation function, while the magnetic field is being applied; and
- measuring MR signals from the imaging volume (30) in order to visualise vessels;
**characterised in that** the RF pulse (40) has a frequency distribution whose frequencies are essentially located on the side of the fat frequency, and which comprises the fat frequency (33) in the imaging volume (30); and
a field distribution of the magnetic field is approximated by a function which is configured in such a way that it has an apex with essentially no spatial gradient in the imaging volume (30) and has a higher spatial gradient in the inflow volume (31), so that the water frequency in the inflow volume (31) is not comprised by the RF pulse.

16. MR system for vascular imaging, wherein the MR system has an MR control unit (13) and a storage unit (16), wherein the storage unit (16) stores control information that can be executed by the MR control unit (13), and wherein the MR system is designed to carry out the method of one of claims 2 to 14 when the control information is executed in the MR control unit (13).

17. Computer program product, which comprises a program, which can be loaded directly into a storage device of an MR control unit (13) of an MR system, having program means in order to carry out the steps of the method according to one of claims 1 to 14 when the program is executed in the MR control unit (13) of the MR system.

18. Electronically readable data carrier with electronically readable control information stored thereon, which is configured in such a way that it carries out the method according to one of claims 1 to 14 when the data carrier is used in an MR control unit (13) of an MR system.

## Revendications

1. Procédé d'imagerie vasculaire reposant sur la technique Time-of-Flight (TOF) à l'aide d'une installation RM, comprenant les stades suivants :
- application d'un champ magnétique à un volume (30) d'imagerie et à un volume (31) d'afflux, dont du liquide entre dans le volume (30) d'imagerie, d'une personne (12, S41) à examiner ;
- excitation du volume (30) d'imagerie par une impulsion (40) HF, qui satisfait une fonction de transfert d'aimantation et une fonction de saturation de graisse pendant que le champ magnétique est appliqué (S42) ; et
- mesure de signaux RM provenant du volume (30) d'imagerie pour l'imagerie vasculaire (S43) ;
**caractérisé en ce que** l'impulsion (40) HF a une répartition de fréquence, dont les fréquences se trouvent sensiblement du côté de la fréquence de la graisse et qui comprennent la fréquence (33) de la graisse dans le volume (30) d'imagerie ; et
**en ce que** le champ magnétique a une répartition du champ magnétique, qui est rendue approximativement par une fonction constituée de manière à avoir une partie de sommet n'ayant sensiblement pas de gradient spatial dans le volume (30) d'imagerie, et un gradient spatial plus grand dans le volume (31) d'afflux de manière à ce que la fréquence de l'eau dans le volume (31) d'afflux ne soit pas comprise par l'impulsion HF.

2. Procédé suivant la revendication 1, dans lequel la fonction est une fonction mathématique au moins du deuxième ordre.

3. Procédé suivant la revendication 1 ou 2, dans lequel le volume (30) d'imagerie et le volume (31) d'afflux sont en forme de couche, un axe longitudinal étant dirigé perpendiculairement au volume (30) d'imagerie et au volume (31) d'afflux et, suivant l'axe longitudinal, une épaisseur de couche du volume (31) d'afflux étant égale à un multiple d'une épaisseur de couche du volume (30) d'imagerie.

4. Procédé suivant l'une des revendications 1 à 3, dans lequel la partie de sommet de la fonction a sensiblement la même épaisseur que l'épaisseur de couche du volume (30) d'imagerie.

5. Procédé suivant l'une des revendications précédentes, dans lequel on effectue les stades du procédé pour au moins un autre volume d'imagerie et au moins un autre volume d'afflux, comprenant, en outre :
- une adaptation de la répartition du champ magnétique sur le au moins un autre volume d'imagerie et le au moins un autre volume d'afflux, de manière à ce que la répartition adaptée du champ magnétique, dans le au moins un autre volume d'imagerie ait la partie de sommet et ait, dans le au moins un autre volume de flux, le gradient spatial plus grand.

6. Procédé suivant la revendication 5, dans lequel l'adaptation de la répartition du champ magnétique comprend un décalage spatial linéaire de la répartition du champ magnétique.

7. Procédé suivant la revendication 5 ou 6, dans lequel l'adaptation de la répartition du champ magnétique comprend une variation d'une composante linéaire de la répartition du champ magnétique.

8. Procédé suivant l'une des revendications 5 à 7, dans lequel les volumes d'imagerie se recoupent dans l'espace au moins en partie.

9. Procédé suivant l'une des revendications précédentes, comprenant, en outre :
- la production de l'impulsion (40) HF par superposition d'une première impulsion (41) HF, qui satisfait une fonction de saturation de graisse, et d'une deuxième impulsion (42) HF, qui satisfait une fonction de transfert d'aimantation.

10. Procédé suivant la revendication 9, dans lequel la première impulsion (41) HF a un premier angle flip et la deuxième impulsion (42) HF a un deuxième angle flip différent du premier angle flip.

11. Procédé suivant la revendication 9 ou 10, dans lequel la première impulsion (41) HF a, de préférence, un angle flip de 20 à 90 degrés, notamment, de préférence, de 90 degrés et une fréquence sensiblement égale à la fréquence (33) de graisse dans le volume (30) d'imagerie.

12. Procédé suivant l'une des revendications précédentes, dans lequel on effectue, par impulsion (40) HF, plusieurs mesures de signaux RM à partir du volume (30) d'imagerie.

13. Procédé suivant la revendication 12, dans lequel on effectue les mesures dans de mêmes intervalles de temps.

14. Procédé suivant la revendication 12, dans lequel on effectue les mesures en regroupements de mesures avec des intervalles de temps entre les diverses mesures, dans lequel on effectue, entre les regroupements de mesures une excitation par une impulsion (40) HF et dans lequel l'intervalle de temps moyen entre les mesures est plus petit qu'une valeur de seuil déterminée à l'avance.

15. Installation RM pour l'imagerie vasculaire, reposant sur la technique Time-of-Fligh (TOF), l'installation RM ayant une unité (13) de commande RM et une unité (16) de mise en mémoire, l'unité (16) de mise en mémoire mémorisant des informations de commande pouvant être réalisées par l'unité (13) de commande RM et l'installation RM étant constituée pour effectuer, lors de la réalisation des informations de commande dans l'unité (13) de commande RM, les stades suivants :
- application d'un champ magnétique à un volume (30) d'imagerie et à un volume (31) d'afflux, dont du liquide entre dans le volume (30) d'imagerie, d'une personne (12) à examiner ;
- excitation du volume (30) d'imagerie par une impulsion (40) HF, qui satisfait une fonction de transfert d'aimantation et une fonction de saturation de graisse pendant que le champ magnétique est appliqué ; et
- mesure de signaux RM provenant du volume (30) d'imagerie pour l'imagerie vasculaire ;
**caractérisé en ce que** l'impulsion (40) HF a une répartition de fréquence, dont les fréquences se trouvent sensiblement du côté de la fréquence de la graisse et qui comprennent la fréquence (33) de la graisse dans le volume (30) d'imagerie ; et
**en ce qu'**une répartition du champ magnétique est rendue approximativement par une fonction constituée de manière à avoir une partie de sommet n'ayant sensiblement pas de gradient spatial dans le volume (30) d'imagerie, et un gradient spatial plus grand dans le volume (31) d'afflux, de manière à ce que la fréquence de l'eau dans le volume (31) d'afflux ne soit pas comprise par l'impulsion HF.

16. Installation RM d'imagerie vasculaire, dans laquelle l'installation RM a une unité (13) de commande RM et une unité (16) de mise en mémoire, l'unité (16) de mise en mémoire mettant en mémoire des informations de commande pouvant être réalisées par l'unité (13) de commande RM et l'installation RM étant constituée pour effectuer, lorsque les informations de commande sont réalisées dans l'unité (13) de commande RM, le procédé suivant l'une des revendications 2 à 14.

17. Produit de programme d'ordinateur, qui comprend un programme, qui peut être chargé directement dans une mémoire d'une unité (13) de commande RM d'une installation RM, comprenant des moyens de programme pour réaliser les stades du procédé suivant l'une des revendications 1 à 14, lorsque le programme est réalisé dans l'unité (13) de commande RM de l'installation RM.

18. Support de données déchiffrables électroniquement, sur lequel se trouvent des informations de commande mises en mémoire et déchiffrables électroniquement, qui sont conformées de manière à être effectuées, lors de l'utilisation du support de données, dans une unité (13) de commande RM d'une installation RM du procédé suivant l'une des revendications 1 à 14.
